# EUROPÄISCHE PATENTSCHRIFT

(11) **EP 1 073 313 B1**
(45) Veröffentlichungstag und Bekanntmachung des Hinweises auf die Patenterteilung: **18.10.2006**
(21) Anmeldenummer: 00105227.3
(22) Anmeldetag: 13.03.2000
(51) Int. Cl.: H04R 25/00

(54) **Anordnung zum mechanischen Ankoppeln eines Treibers an eine Ankoppelstelle der Ossikelkette**
Device for mechanical coupling of a driver to a coupling part of the ossicular chain
Dispositif pour le couplage mécanique d'un vibreur à une place de couplage de la chaîne ossiculaire

(30) Priorität: 26.07.1999 DE 19935029
(43) Veröffentlichungstag der Anmeldung: 31.01.2001
(73) Patentinhaber: PHONAK AG, 8712 Stäfa (CH)
(72) Erfinder: Müller, Gerd M., Dr.rer.nat.Dr.Dipl.-Phys., 85716 Lohhof (DE); Leysieffer, Hans, Dr.-Ing. Diplom-Ingenieur, 82024 Taufkirchen (DE)
(74) Vertreter: Schwan, Gerhard

(56) Entgegenhaltungen:
- WO-A-99/04600
- WO-A-99/15111

## Beschreibung

Die Erfindung betrifft eine implantierbare Anordnung zum mechanischen Ankoppeln eines zu mechanischen Schwingungen anregbaren ausgangsseitigen Treiberteils eines aktiven oder passiven Hörsystems an eine vorgewählte Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran über eine Koppelanordnung, die ein mit der vorgewählten Ankoppelstelle in, vorzugsweise formschlüssige, Verbindung bringbares Koppelelement aufweist.

Es sind teil- oder vollimplantierbare aktive Hörsysteme für eine direkte mechanische Stimulation bekannt. Bei solchen Hörsystemen wird das Schallsignal mit einem Sensor (Mikrofon) in ein elektrisches Signal umgewandelt und in einer elektronischen Signalverarbeitungsstufe verstärkt; dieses verstärkte elektrische Signal wird einem implantierten elektromechanischen Wandler zugeführt, dessen ausgangsseitige mechanische Schwingungen unmittelbar, also mit direktem mechanischen Kontakt, dem Mittel- beziehungsweise Innenohr zugeführt werden. Dies gilt unabhängig davon, ob eine reine Innenohrschwerhörigkeit bei vollständig intaktem Mittelohr oder eine kombinierte Schwerhörigkeit (Mittel- und Innenohr geschädigt) rehabilitiert werden soll. Daher sind in der jüngeren wissenschaftlichen und Patent-Literatur implantierbare elektromechanische Wandler sowie Verfahren zur direkten Ankopplung der mechanischen Wandlerschwingungen an das intakte Mittelohr beziehungsweise das Innenohr zur Rehabilitation einer reinen Innenohrschwerhörigkeit sowie auch an verbleibende Ossikel des Mittelohres bei artifiziell oder pathologisch verändertem Mittelohr zur Versorgung einer Schalleitungsschwerhörigkeit sowie deren Kombinationen beschrieben worden.

Als elektromechanisches Wandlerverfahren kommen grundsätzlich alle physikalischen Wandlungsprinzipien in Frage wie elektromagnetisch, elektrodynamisch, magnetostriktiv, dielektrisch und piezoelektrisch. Verschiedene Forschungsgruppen haben sich in den letzten Jahren im wesentlichen auf zwei dieser Verfahren konzentriert: elektromagnetisch und piezoelektrisch. Eine Übersicht über diese Wandlervarianten findet sich bei Zenner und Leysieffer (HNO 1997 Vol. 45, 749 - 774).

Beim piezoelektrischen Verfahren ist eine mechanisch direkte Kopplung der ausgangsseitigen Wandlerschwingungen an die Mittelohrossikel oder direkt an das ovale Fenster notwendig. Beim elektromagnetischen Prinzip kann die Kraftkopplung einerseits über einen Luftspalt erfolgen ("kontaklos"), das heißt, nur ein Permanentmagnet wird durch dauerhafte Fixation in direkten mechanischen Kontakt mit einem Mittelohrossikel gebracht. Andererseits besteht die Möglichkeit, den Wandler vollständig in einem Gehäuse zu realisieren (wobei Spule und Magnet mit kleinstmöglichem Luftspalt gekoppelt sind) und die ausgangsseitigen Schwingungen über ein mechanisch steifes Koppelelement mit direktem Kontakt auf die Mittelohrossikel zu übertragen (Leysieffer et al. 1997 (*HNO 1997, Vol. 45, pp. 792-800*).

In der Patentliteratur finden sich einige der oben genannten Realisierungsvarianten sowohl elektromagnetischer wie auch piezoelektrischer Hörgerätewandler: US-A-5 707 338 (Adams et al.), WO 98/06235 (Adams et al.), WO 98/06238 (Adams et al.), WO 98/06236 (Kroll et al.), WO 98/06237 (Bushek et al.), US-A-5 554 096 (Ball), US-A-3 712 962 (Epley), US-A-3 870 832 (Fredrickson), US-A-5 277 694 (Leysieffer et al.), EP-A-0 984 663 (Leysieffer), EP-A-0 984 665 (Leysieffer), US-A-5 015 224 (Maniglia), US-A-3 882 285 (Nunley), US-A-4 850 962 (Schaefer).

Das teilimplantierbare, piezoelektrische Hörsystem der japanischen Gruppe um Suzuki und Yanigahara setzt für eine Implantation des Wandlers das Fehlen der Mittelohrossikel und eine freie Paukenhöhle voraus, um das Piezoelement an den Stapes ankoppeln zu können (YANIGAHARA et al.: *Efficacy of the partially implantable middle ear implant in middle and inner ear disorders*. Adv. Audiol.., Vol. 4, Karger Basel (1988), pp. 149-159. SUZUKI et al.: *Implantation of partially implantable middle ear implant and the indication*. Adv. Audiol., Vol. 4, Karger Basel (1988), pp. 160-166). Ebenso wird bei dem Verfahren eines implantierbaren Hörsystems für Innenohrschwerhörige nach US-A-4 850 962 (Schaefer) grundsätzlich der Amboß entfernt, um ein piezoelektrisches Wandlerelement an den Stapes ankoppeln zu können. Dies gilt im wesentlichen auch für weitere Entwicklungen, die auf der SCHAEFER-Technologie basieren und in den oben genannten Patentschriften dokumentiert sind (US-A-5 707 338, WO 98/06235, WO 98/06238, WO 98/06236, WO 98/06237).

Der elektromagnetische Wandler nach Ball ("*Floating Mass Transducer FMT*", US-A-5 624 376, US-A-5 554 096) wird dagegen bei intaktem Mittelohr mit Titanclips direkt an dem langen Fortsatz des Amboß fixiert. Der elektromagnetische Wandler des teilimplantierbaren Systems nach FREDRICKSON (FREDRICKSON et al.: *Ongoing investigations into an implantable elektromagnetic hearing aid for moderate to severe sensorineural hearing loss*. Otolaryngologic Clinics Of Nord America, Vol. 28/1 (1995), pp. 107-121) wird bei ebenfalls intakter Ossikelkette des Mittelohres mechanisch direkt an den Amboßkörper gekoppelt. Das Gleiche gilt für die piezoelektrischen und elektromagnetischen Wandler nach LEYSIEFFER (Leysieffer et al.: *Ein implantierbarer piezoelektrischer Hörgerätewandler für Innenohrschwerhörige*. HNO 1997/45, pp. 792-800, US-A-5 277 694, EP-A-0 984 663, EP-A-0 984 665). Auch bei dem elektromagnetischen Wandlersystem nach MANIGLIA (Maniglia et al.: *Contactless semi-implantable electromagnetic middle ear device for the treatment of sensorineural hearing loss*. Otolaryngologic Clinics Of Nord America, Vol. 28/1 (1995), pp. 121-141) wird bei intakter Ossikelkette ein Permanentmagnet mechanisch an der Ossikelkette dauerhaft fixiert, der jedoch über eine Luftspaltkopplung von einer Spule mechanisch angetrieben wird.

Bei den beschriebenen Wandler- und Ankopplungsvarianten sind grundsätzlich zwei Implantationsprinzipien zu unterscheiden:
a) Einerseits befindet sich der elektromechanische Wandler mit seinem aktiven Wandlerelement selbst im Mittelohrbereich in der Paukenhöhle und ist dort direkt mit einem Ossikel oder dem Innenohr verbunden (US-A-4 850 962, US-A-5 015 225, US-A-5 707 338, WO 98/06235, WO 98/06238, WO 98/06236, WO 98/06237, US-A-5 624 376, US-A-5 554 096).
b) Andererseits befindet sich der elektromechanische Wandler mit seinem aktiven Wandlerelement außerhalb des Mittelohrbereiches in einer artifiziell geschaffenen Mastoidhöhle; die ausgangsseitigen mechanischen Schwingungen werden dann mittels mechanisch passiver Koppelelemente über geeignete operative Zugänge (natürlicher aditus ad antrum, Eröffnung des chorda-facialis-Winkels oder über eine artifizielle Bohrung vom Mastoid aus) zum Mittel- beziehungsweise Innenohr übertragen (FREDRICKSON et al.: *Ongoing investigations into an implantable elektromagnetic hearing aid for moderate to severe sensorineural hearing loss*. Otolaryngologic Clinics Of Nord America, Vol. 28/1 (1995), pp. 107-121; US-A-5 277 694; EP-A-0 984 663; EP-A-0 984 665).

Bei den Varianten nach a) kann der Wandler als sogenannter "Floating Mass"-Wandler ausgeführt sein, d.h., das Wandlerelement benötig keine "Reaktio" über eine feste Verschraubung mit dem Schädelknochen, sondern es schwingt aufgrund von Massenträgheitsgesetzen mit seinem Wandlergehäuse und überträgt diese direkt auf ein Mittelohrossikel (US-A-5 624 376, US-A-5 554 096, US-A-5 707 338, WO 98/06236). Dies bedeutet einerseits, daß vorteilhaft auf ein implantierbares Fixationssystem an der Schädelkalotte verzichtet werden kann; andererseits bedeutet diese Variante nachteilig, daß voluminöse artifizielle Elemente in die Paukenhöhle eingebracht werden müssen und deren Langzeit- und Biostabilität insbesondere bei temporären pathologischen Veränderungen des Mittelohres (z.B. otitis media) heute nicht bekannt beziehungsweise gewährleistet sind. Ein weiterer wesentlicher Nachteil besteht darin, daß die Wandler vom Mastoid aus mit ihrer elektrischen Zuleitung ins Mittelohr gebracht und dort mit Hilfe geeigneter operativer Werkzeuge fixiert werden müssen; dies erfordert einen erweiterten Zugang durch den chorda-facialis-Winkel und bringt somit eine latente Gefährdung des in unmittelbarer Nachbarschaft gelegenen Gesichtsnerven (nervus facialis) mit sich.

Bei den Wandlervarianten nach b) wird das Wandlergehäuse mit implantierbaren Positionier- und Fixationssystemen an der Schädelkalotte befestigt (vorteilhafte Ausführung DE-A-196 18 964 entsprechend US-A-5 788 711). Sowohl bei dem teilimplantierbaren System nach FREDRICKSON (*Ongoing investigations into an implantable elektromagnetic hearing aid for moderate to severe sensorineural hearing loss*. Otolaryngologic Clinics Of Nord America, Vol. 28/1 (1995), pp. 107-121) wie auch bei dem vollimplantierbaren Hörsystem nach LEYSIEFFER und ZENNER (HNO 1998, Vol. 46, 853-863 und 844-852) wird bei der Ankopplung des schwingenden Treiberteils an den Amboßkörper zur dauerhaften und mechanisch sicheren Schwingungsübertragung davon ausgegangen, daß die Spitze der Koppelstange, die in die laserinduzierte Vertiefung des Mittelohrossikels eingebracht wird, langfristig eine Osseointegration erfährt, das heißt, die Koppelstange verwächst fest mit dem Ossikel und gewährleistet so eine sichere Übertragung dynamischer Druck- und Zugkräfte. Dieser Langzeiteffekt ist zur Zeit jedoch noch nicht wissenschaftlich nachgewiesen beziehungsweise gesichert. Weiterhin besteht bei dieser Ankopplungsart bei einem technischen Wandlerdefekt der Nachteil, daß eine Entkopplung vom Ossikel zur Entfernung des Wandlers nur mit mechanisch basierten operativen Methoden vorgenommen werden kann, was eine erhebliche Gefährdung des Mittelohres und insbesondere des Innenohres bedeuten kann.

Der wesentliche Vorteil dieser Wandlerausführungsformen nach b) besteht jedoch darin, daß das Mittelohr weitgehend frei bleibt und der Koppelzugang zum Mittelohr ohne größeres Gefährdungspotential des nervus facialis erfolgen kann. Ein vorzugsweises operatives Verfahren hierzu ist in der US-Patentanmeldung 09/168.079 beschrieben. Grundlegende vorteilhafte Formen passiver Koppelelemente zur Übertragung der ausgangsseitigen Wandlerschwingungen vom Mastoid aus zum Mittel- beziehungsweise Innenohr sind in EP-A-0 499 940 (entsprechend US-A-5 277 964) sowie in EP-A-0 901 779 (entsprechend US-A-5 941 814) und in HNO 1998 Vol. 46, 27 - 37 - Lehner et al.: "Kaltfließende Elemente zur Ankopplung eines implantierbaren Hörgerätewandlers an Gehörknöchelchen oder Perilymphe" beschrieben. Dabei handelt es sich insbesondere um Koppelelemente aus Gold, vorzugsweise weichgeglühtem Feingold, in Form eines C-Bandes für den langen Amboßfortsatz, einer Bandschlaufe für den langen Amboßfortsatz und eines Glöckchens für das Steigbügelköpfchen, wobei sich diese Koppelelemente unter Verwendung von ohrchirurgischen Standardinstrumenten ankoppeln und erforderlichenfalls auch wieder lösen lassen.

Bei aktiven wie passiven Hörsystemen wird eine große Übertragungsbandbreite angestrebt, um neben Sprachsignalen beispielsweise auch Musiksignale getreu übermitteln zu können. Dabei spielt neben den spektralen Anforderungen an das Hörsystem auch das dynamische Betriebsverhalten eine wichtige Rolle, wenn es darum geht, den zeitlichen Hüllkurvenverlauf eines Audiosignals insbesondere bei stark veränderlichen Signalanteilen wie plosiven Lauten bei der Sprachdiskrimination und bei impulshaltigen Anteilen bei Musikübertragung möglichst naturgetreu wiederzugeben. Des weiteren wird in der Regel für aktive Hörsysteme eine frequenzunabhängige mechanische Auslenkung des ausgangsseitigen Treiberteils und damit verbundener Koppelglieder bei konstanter Wandlerspannung des elektromechanischen Wandlers angestrebt. Bekannte Koppelglieder im Schwingungsübertragungsweg zwischen dem ausgangsseitigen Treiberteil und der vorgewählten Ankoppelstelle, beispielsweise in Form von metallischen oder keramischen Koppelstangen eines aktiven Hörsystems oder von aus biokeramischen Werkstoffen hergestellten Prothesen eines passiven Hörsystems, weisen typischerweise eine hohe Resonanzüberhöhung (mechanische Güte) auf; es kommt zu einer schallharten Ankopplung. Eine hohe mechanische Güte kann auch auf Seiten des elektromechanischen Wandlers vorliegen. Infolgedessen kann es innerhalb des breiten Übertragungsbereiches zu unerwünschten Resonanzerscheinungen kommen. Dies gilt insbesondere bei Unterbringung des elektromechanischen Wandlers in der Mastoidhöhle, weil bei der in diesem Fall vorliegenden Relation der Größe des massebehafteten Koppelgliedes zum Volumen der dynamischen Anteile des aktiven Wandlerelements mit einem deutlichen Einfluß des Koppelgliedes auf die dynamischen Eigenschaften und damit auf die gesamte Übertragungsfunktion des Wandlersystems gerechnet werden muß. Es ist bekannt (WO 99/15111), für eine Dämpfung im Übertragungsweg zwischen einem aktiven Wandlerelement eines aktiven Hörsystems und einer Ankoppelstelle an der Ossikelkette dadurch zu sorgen, daß zwischen das Wandlerelement und ein mit der Ankoppelstelle in Antriebsverbindung zu bringendes Koppelelement ein nachgiebiges Verbindungsteil, beispielsweise in Form einer Feder oder eines Urethanstreifens, eingefügt wird. Das zwischen dem Koppelelement und dem aktiven Wandlerelement sitzende nachgiebige Verbindungsteil sorgt für eine elastische Kopplung des aktiven Wandlerelements mit dem Koppelelement. Es ist ferner bekannt (Alaa El SEIFI: *The Necrosed Incus in Stapedectomy Revision*, Laryngoscope 106, April 1996, pp. 511-512) in Fällen, bei denen sich eine zum Ankoppeln einer passiven Stapesprothese an einen Incusstumpf vorgesehene Drahtschlinge gelockert hat, ein über einen Teil seiner Längsabmessung geschlitztes Schlauchstück aus Silastic® über die Drahtschlinge und den Incusstumpf zu schieben, um auf diese Weise die Drahtschlinge gegen den Incusstumpf zu drücken und die Kopplung zwischen beiden wiederherzustellen.

Der vorliegenden Erfindung liegt die Aufgabe zugrunde, eine implantierbare Anordnung zum mechanischen Ankoppeln eines zu mechanischen Schwingungen anregbaren ausgangsseitigen Treiberteils eines aktiven oder passiven Hörsystems zu schaffen, die sich durch vorteilhafte Eigenschaften für die Übertragung der Schwingungen von dem ausgangsseitigen Treiberteil zu der vorgewählten Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran auszeichnet, die eine optimale Schwingungsform der Steigbügelfußplatte beziehungsweise der genannten Membran begünstigt und die das Risiko einer Beschädigung der natürlichen Strukturen im Bereich der Ankoppelstelle während und nach der Implantation besonders gering hält.

Ausgehend von einer Vorrichtung der eingangs genannten Art, das heißt einer implantierbaren Anordnung zum mechanischen Ankoppeln eines zu mechanischen Schwingungen anregbaren ausgangsseitigen Treiberteils eines aktiven oder passiven Hörsystems an eine vorgewählte Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran über eine Koppelanordnung, die ein mit der vorgewählten Ankoppelstelle in Verbindung bringbares Koppelelement aufweist, wird diese Aufgabe erfindungsgemäß gelöst durch ein im implantierten Zustand an der Ankoppelstelle anliegendes Dämpfungsglied mit entropieelastischen Eigenschaften.

Unter einem Dämpfungsglied mit entropieelastischen Eigenschaften soll dabei ein Dämpfungsglied verstanden werden, das mindestens eine gewisse Entropieelastizität, gegebenenfalls in Kombination mit Energie- oder Stahlelastizität (siehe dazu DIN 7724), aufweist. Entropieelastizität, (auch Gummielastizität genannt) bedeutet, daß eine elastische Verformung im wesentlichen ohne Änderung der inneren Energie abläuft. Entropieelastizität ist in dem Bestreben großer Makromoleküle begründet, eine möglichst ungeordnete Form anzunehmen.

Die erfindungsgemäße Anordnung sorgt auf besonders einfache und gleichwohl zuverlässige Weise für einen relativ ebenen Frequenzgang für die Auslenkungen, die an der vorgewählten Ankoppelstelle in Abhängigkeit von Schwingungen des ausgangsseitigen Treiberteils auftreten. Ein weiterer wichtiger Vorteil besteht darin, daß die Ankoppelstelle zum Beispiel des Ossikels nicht hauptsächlich in Schwingungsrichtung des antreibenden Wandlers "zwangsgeführt" wird, wobei eine solche "Zwangsführung" zu einer nichtoptimalen Schwingungsform der Steigbügelfußplatte im ovalen Fenster führen kann. (Eine vorzugsweise Schwingungsform ist eine kolbenförmige Schwingung der Steigbügelfußplatte senkrecht zu ihrer Ebene.) Vielmehr stellt das Ossikel aufgrund der Nachgiebigkeit des Dämpfungsgliedes seine (frequenzabhängige) Schwingungsrichtung aufgrund der dynamischen Eigenschaften des intakten Mittelohres selbst ein. Dieser Vorteil gilt auch bei nicht-intakter, (teil-)abgebauter Ossikelkette und Ankopplung an den dem Innenohr zugewandten "Rest" der Kette, und im Extremfall auch bei nur verbleibendem Steigbügel oder nur Steigbügelfußplatte, da diese(r) durch das sogenannte Ligament (ein elastisches Ringband, das den Steigbügel im ovalen Fenster "hält") aufgehängt ist. Zugleich schützt das Dämpfungsglied die Ankoppelstelle auf wirkungsvolle Weise gegen Beschädigung durch das Koppelelement sowohl während als auch nach der Implantation.

In weiterer Ausgestaltung der Erfindung kann das Dämpfungsglied im implantierten Zustand mindestens zum Teil zwischen dem Koppelelement und der Ankoppelstelle liegen. Dabei kann das Dämpfungsglied vorteilhaft als Formteil ausgebildet sein, das im implantierten Zustand einen Teil der Ossikelkette mindestens teilweise umgreift. Als Dämpfungsglied kann aber auch eine Beschichtung mit entropieelastischen Eigenschaften auf der mit der Ankoppelstelle in Berührung kommenden Seite des Koppelelements vorgesehen sein.

Entsprechend einer abgewandelten Ausführungsform der Erfindung weist der Schwingungsübertragungsweg eine mit dem ausgangsseitigen Treiberteil in Antriebsverbindung stehende Koppelstange und ein an der Koppelstange befestigtes, mit der vorgewählten Ankoppelstelle in Antriebsverbindung bringbares Koppelelement auf, wobei das Dämpfungsglied als Beschichtung mit entropieelastischen Eigenschaften ausgebildet ist, welche das Koppelelement mindestens teilweise umschließt, und wobei im implantierten Zustand ein Teil der Beschichtung zwischen dem Koppelelement und der Ankoppelstelle liegt.

Bei einer weiter abgewandelten Ausführungsform der Erfindung weist der Schwingungsübertragungsweg eine mit dem ausgangsseitigen Treiberteil in Antriebsverbindung stehende Koppelstange und ein mit der Koppelstange verbundenes, mit der vorgewählten Ankoppelstelle in Antriebsverbindung bringbares Koppelelement auf, und das Dämpfungsglied sitzt zwischen der Koppelstange und dem Koppelelement. Dabei kann zweckmäßig das Dämpfungsglied als Verbindungselement zwischen der Koppelstange und dem Koppelelement ausgebildet sein. Insbesondere kann als Dämpfungsglied ein im wesentlichen zylindrisches Formteil vorgesehen sein, das mit Aufnahmen für das koppelelementseitige Ende der Koppelstange und für das koppelstangenseitige Ende des Koppelelements versehen ist. Die Ausbildung kann aber auch so gestaltet sein, daß das koppelstangenseitige Ende des Koppelelements das koppelelementseitige Ende der Koppelstange unter Bildung eines Spaltes zwischen diesen beiden Enden umgreift und der Spalt mit einem das Dämpfungsglied bildenden Material mit entropieelastischen Eigenschaften mindestens teilweise gefüllt ist.

Gemäß einer weiteren Ausgestaltung der Erfindung ist das Koppelelement selbst als Dämpfungsglied ausgebildet. So kann als Koppelelement und Dämpfungsglied ein Formteil mit entropieelastischen Eigenschaften vorgesehen sein, das mit einem freien Ende in den Raum zwischen Steigbügel und Steigbügelfußplatte einschiebbar ist.

Als Werkstoff für das Dämpfungsglied eignen sich insbesondere vernetzte Silicone oder ein anderer implantierbarer, kautschukartiger Werkstoff.

Die erfindungsgemäße Anordnung kann Teil eines aktiven Hörsystems sein, bei dem das ausgangsseitige Treiberteil ein schwingfähiges Teil, insbesondere eine schwingfähige Membran, eines elektromechanischen Hörgerätewandlers ist. Die Anordnung nach der Erfindung kann aber auch Teil eines passiven Hörsystems, insbesondere einer Teil- oder Voll-Mittelohrprothese, sein, bei dem im implantierten Zustand das Trommelfell als ausgangsseitiges Treiberteil genutzt ist.

Bevorzugte Ausführungsbeispiele der Anordnung nach der Erfindung werden nachstehend anhand der beiliegenden Zeichnungen näher erläutert. Es zeigen:
- Fig. 1: in größerem Maßstab schematisch einen implantierten Hörgerätewandler mit an die Ossikelkette angekoppelter Koppelstange,
- Fig. 2: in noch größerem Maßstab eine perspektivische Darstellung eines mit der Koppelstange des Hörgerätewandlers nach Fig. 1 über ein Dämpfungsglied verbundenen Koppelelements zum Ankoppeln des Hörgerätewandlers an den Amboßkörper,
- Fig. 3: eine perspektivische Darstellung des Koppelelements nach Fig. 2 im implantierten Zustand,
- Fig. 4: einen Längsschnitt des Dämpfungsgliedes nach den Figuren 2 und 3,
- Fig. 5: eine perspektivische Darstellung einer als Koppelelement vorgesehenen Federklammer, die mit einer Koppelstange über ein Dämpfungsglied gemäß Fig. 4 verbunden ist,
- Fig. 6: eine auseinandergezogene perspektivische Darstellung einer Anordnung zum Ankoppeln an den Amboßschenkel unter Verwendung eines Dämpfungsgliedes in Form eines längsgeschlitzten Schlauchstückes,
- Fig. 7: eine perspektivische Darstellung der Anordnung nach Fig. 6 im angekoppelten Zustand,
- Fig. 8: eine perspektivische Darstellung eines Dämpfungsgliedes in Form einer längsgeschlitzten Muffe,
- Fig. 9: einen Längsschnitt des Dämpfungsgliedes nach Fig. 8 in Verbindung mit einer Federklammer gemäß Fig. 10,
- Fig. 10: eine perspektivische Darstellung des Dämpfungsgliedes nach Fig. 8 und einer als Koppelelement vorgesehenen Federklammer,
- Fig. 11: einen Längsschnitt eines mit einer Koppelstange über eine abgewandelte Ausführungsform eines Dämpfungsgliedes verbundenen Koppelelements,
- Fig. 12: eine perspektivische Darstellung der Anordnung nach Fig. 11,
- Fign. 13 und 14: einen Längsschnitt und eine perspektivische Darstellung eines Koppelelements in Form einer Federklammer mit einer als Dämpfungsglied wirkenden Beschichtung,
- Fign. 15 bis 17: eine abgewandelte Federklammer mit einer Beschichtung entsprechend den Figuren 13 und 14 bei unterschiedlicher Anbringung an einer Koppelstange,
- Fign. 18 und 19: einen Längsschnitt und eine perspektivische Darstellung eines Koppelelements in Form einer Federklammer mit einer als Dämpfungsglied wirkenden Umhüllung,
- Fig. 20: eine perspektivische Darstellung eines als Dämpfungsglied ausgebildeten Koppelelements,
- Fig. 21 und 22: perspektivische Darstellungen zweier weiterer Ausführungsbeispiele von Koppelelementen mit Dämpfungsgliedern, sowie
- Fign. 23 bis 25: perspektivische Darstellungen von Ausführungsformen, bei denen das Koppelelement selbst als Dämpfungsglied ausgebildet ist,
- Fign. 26 bis 31: Darstellungen von Ausführungsformen, bei denen das Koppelelement nebst Dämpfungsglied mit einer Koppelstange über ein Kugelgelenk verbunden ist, und
- Fig. 32: eine schematische perspektivische Darstellung einer erfindungsgemäßen passiven Mittelohrprothese.

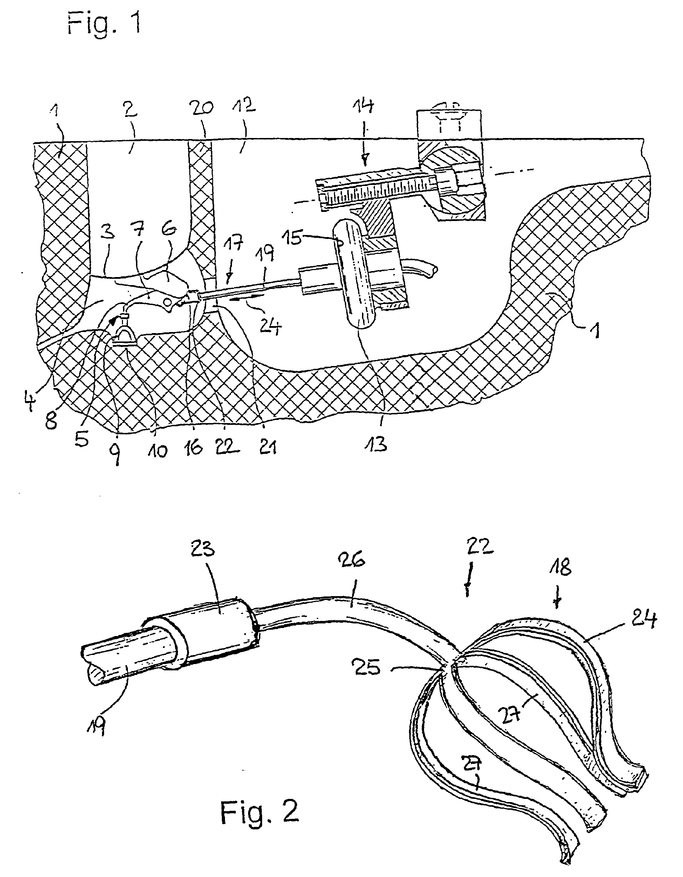

In Fig. 1 ist ein Teil eines menschlichen Schädelknochens 1 mit dem Gehörgang 2, dem davon durch das Trommelfell 3 abgetrennten Mittelohrraum (Paukenhöhle) 4 und der in der Paukenhöhle befindlichen Ossikelkette 5 dargestellt. Zu der Ossikelkette 5 gehören der Hammer 6, der Amboß 7 mit dem langen Amboßfortsatz 8 sowie der Steigbügel 9 mit der Steigbügelfußplatte 10. In einer artifiziellen Mastoidhöhle 12 ist ein elektromechanischer Hörgerätewandler 13 mittels eines insgesamt mit 14 bezeichneten Positionier- und Fixiersystems fixiert. Der Hörgerätewandler 13 kann beispielsweise als Piezowandler zur vibratorischen Stimulation der Ossikelkette insbesondere in der aus US-A-5 277 694 bekannten Weise aufgebaut sein, und er ist Bestandteil eines mindestens teilimplantierbaren und vorzugsweise vollimplantierbaren Hörgerätes, beispielsweise eines Hörgerätes der aus HNO 1997 Vol. 45, 749 - 774 bekannten Art.

Zum mechanischen Ankoppeln eines zu mechanischen Schwingungen anregbaren, in Fig. 1 nur schematisch angedeuteten ausgangsseitigen Treiberteils 15 des Hörgerätewandlers 13, insbesondere einer schwingfähigen Membran dieses Wandlers, an eine vorgewählte Ankoppelstelle 16 an der Ossikelkette 5, beispielsweise an den "glatten" Körper des Ambosses 7, von Mastoidseite aus, ist ein Schwingungsübertragungsweg in Form einer biokompatiblen, mechanisch passiven Koppelanordnung 17 vorgesehen. Die Koppelanordnung 17 ist mit dem aktiv schwingfähigen ausgangsseitigen Treiberteil 15 verbunden, und sie liegt im implantierten Zustand mit einem von dem Hörgerätewandler 13 abliegenden Ankoppelende an der Ankoppelstelle 16 an. Wird an den Hörgerätewandler 13 eine elektrische Spannung angelegt, wird die Koppelanordnung 17 mittels des ausgangsseitigen Treiberteils 15 zu vibratorischen Schwingungen in Axialrichtung der Koppelanordnung veranlaßt. Infolgedessen führen die von einem (nicht dargestellten) eingangsseitigen Wandler (Mikrofon) aufgenommenen und elektrisch gewandelten Audiosignale nach elektronischer Verstärkung in einem Elektronikmodul des aktiven Hörsystems unmittelbar zu mechanischen Auslenkungen der Koppelanordnung 17. Diese Auslenkungen entsprechen der akustischen Information. Die Auslenkungen der Koppelanordnung 17 werden an die Ossikelkette 5 des Mittelohrs bzw. an den Steigbügel 9, die Steigbügelfußplatte 10 oder eine nicht dargestellte Membran weitergeleitet, die das ovale beziehungsweise runde Fenster oder ein artifizielles Fenster in der Cochlea, im Vestibulum oder im Labyrinth (Gleichgewichtsorgan) abschließt. Die Auslenkungen der Koppelanordnung bewirken so bei entsprechender Auslegung des vorverarbeitenden elektronischen Systems einen audiologischen Verstärkungseffekt.

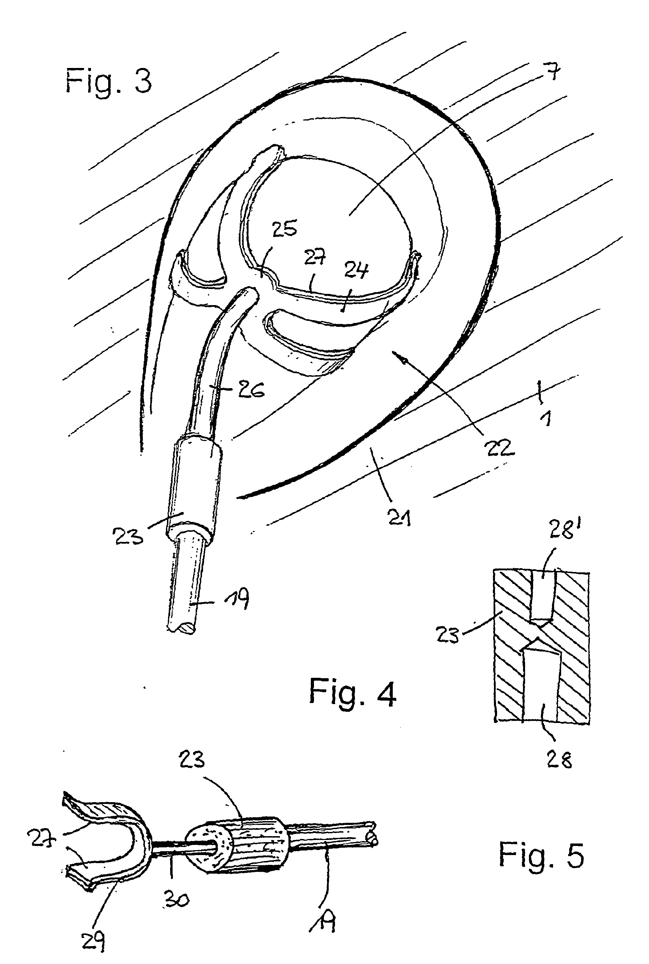

Die Koppelanordnung 17 weist im veranschaulichten Ausführungsbeispiel eine mit dem ausgangsseitigen Treiberteil 15 mechanisch fest verbundene Koppelstange 19 auf, die bei der gezeigten Ausführungsform im wesentlichen auf ihrer gesamten Länge die Gestalt eines geraden Zylinders hat. Die Koppelstange 19 reicht im implantierten Zustand von der Mastoidhöhle 12 aus bevorzugt durch einen in der hinteren Gehörgangswand 20 befindlichen natürlichen, erforderlichenfalls artifiziell erweiterten Knochendurchbruch (Aditus ad antrum) 21 hindurch in die Paukenhöhle 4. Zu der Koppelanordnung 17 gehört ferner ein im einzelnen in den Figuren 2 und 3 veranschaulichtes Koppelelement 22, das mit dem von dem Hörgerätewandler 13 abliegenden Ende der Koppelstange 19 über eine Kupplung 23 verbunden ist und ein Ankoppelende 18 der Koppelanordnung 17 bildet. Das Koppelelement 22 weist bei dieser Ausführungsform am Ankoppelende 18 vier Federarme 24 auf, die sich mit ihrem jeweils einen Ende an einem Anschlußstück 25 treffen. Letzteres ist mit einem Stiel 26 verbunden.

Als zwischen dem Koppelelement 22 und der Ankoppelstelle 16 liegendes Dämpfungsglied 27 ist eine Beschichtung mit entropieelastischen Eigenschaften vorgesehen. Die Beschichtung befindet sich auf der Seite der Federarme 24, die im implantierten Zustand mit der Ankoppelstelle 16 in Berührung kommt. Das Koppelelement 22 faßt über das Dämpfungsglied 27 den Körper des Ambosses 7, so daß es zu einer dynamischen Zug-Druck-Kraftkopplung von Koppelelement 22 und Zielossikel (in dem veranschaulichten Fall dem Amboß 7) kommt. Das Dämpfungsglied 27 besteht aus einem entropie- oder gummielastischen Werkstoff, vorzugsweise einem vernetzten Silicon. Es senkt die mechanische Güte des Schwingungsübertragungsweges 17 und erhöht damit die Ebenheit des Auslenkungsfrequenzganges des Hörsystems. Zugleich schützt das Dämpfungsglied 27 die Ankoppelstelle 16 gegen Beschädigung durch die vorzugsweise metallischen Federarme 24 beim und nach dem Implantieren.

Die in größerem Maßstab in Fig. 4 gezeigte Kupplung 23 ist ein im wesentlichen zylindrisches Formteil. Es weist zwei miteinander ausgerichtete, axial verlaufende Ausnehmungen 28, 28' zur Aufnahme des koppelelementseitigen Endes der Koppelstange 19 und des koppelstangenseitigen Endes des Stiels 26 des Koppelelements 22 auf. Diese Enden sind im implantierten Zustand mit der Kupplung 23 fest verbunden. Beispielsweise können sie in die Ausnehmungen 28, 28' unter Herstellung einer Kraftschlußverbindung eingepreßt und/oder eingeklebt sein.

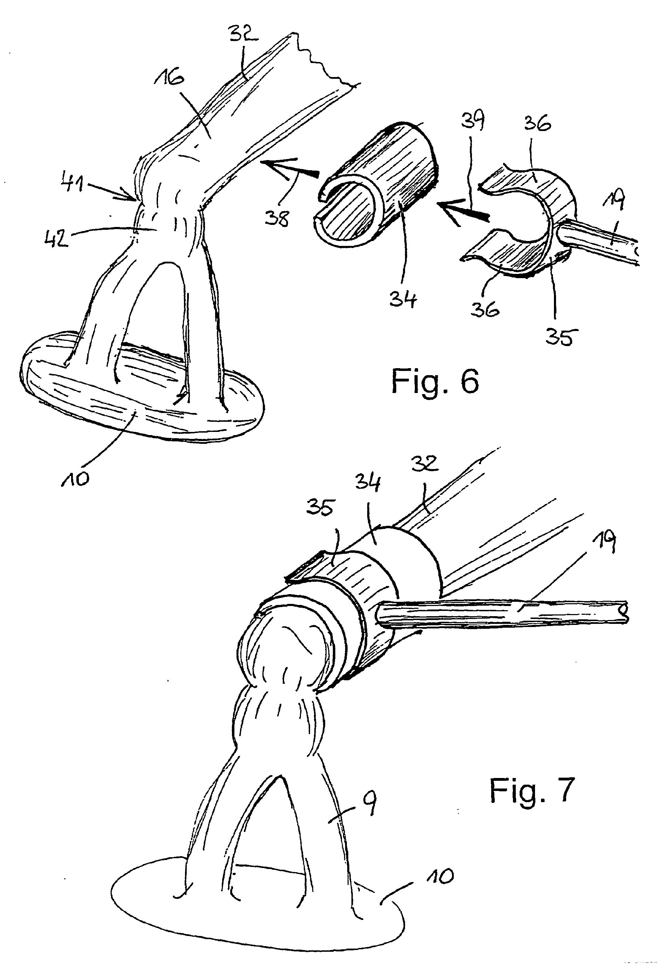

Bei der Ausführungsform der Fig. 5 ist das Koppelelement 29 als einen Stiel 30 aufweisende zweiarmige Federklammer ausgebildet, die beispielsweise zur Ankopplung an den langen Amboßschenkel 32 (Fig. 6) geeignet ist. Auch in diesem Fall dient die Kupplung 23 als Verbindungselement zwischen der Koppelstange 19 und dem Koppelelement 29, wobei die freien Enden von Koppelstange 19 und Stiel 30 in die Ausnehmungen 28, 28' der Kupplung 23 eingesteckt sind. Das Koppelelement 29 trägt auf seiner der Ankoppelstelle 16 zugewendeten Seite eine als Dämpfungsglied 27 wirkende Beschichtung analog der zuvor erläuterten Beschichtung der Federarme 24.

Die Figuren 6 und 7 zeigen ein Ausführungsbeispiel, bei dem ein Dämpfungsglied 34 als Formteil ausgebildet ist, das im implantierten Zustand zwischen der Ankoppelstelle 16 und dem Koppelelement 35 liegt und einen Teil der Ossikelkette, beispielsweise den langen Amboßschenkel 32, mindestens teilweise umgreift. Das Koppelelement 35 entspricht in diesem Beispiel dem Koppelelement 29 der Fig. 5 mit der Ausnahme, daß die mit zwei Federarmen 36 versehene Federklammer unmittelbar an der Koppelstange 19 befestigt ist und die Federarme 36 keine Beschichtung 27 aufweisen. Bei dem das Dämpfungsglied 34 bildenden Formteil handelt es sich um ein längsgeschlitztes Schlauchstück aus entropie- oder gummielastischem Werkstoff, vorzugsweise einem Siliconharz. Bei der Implantation wird zunächst das Dämpfungsglied 34 auf den betreffenden Teil der Ossikelkette aufgesteckt (Pfeil 38 in Fig. 6). Dann wird das Koppelelement 35 durch Vorbewegen der Koppelstange 19 auf das Dämpfungsglied 34 aufgeschoben (Pfeil 39 in Fig. 6), wobei das Dämpfungsglied 34 zugleich die Ankoppelstelle 16 gegen Beschädigung durch die Federarme 36 des Koppelelements 35 schützt.

In den Figuren 6 und 7 sind das Incudo-Stapedialgelenk bei 41, der Steigbügelkopf bei 42 und die Steigbügelfußplatte bei 10 angedeutet.

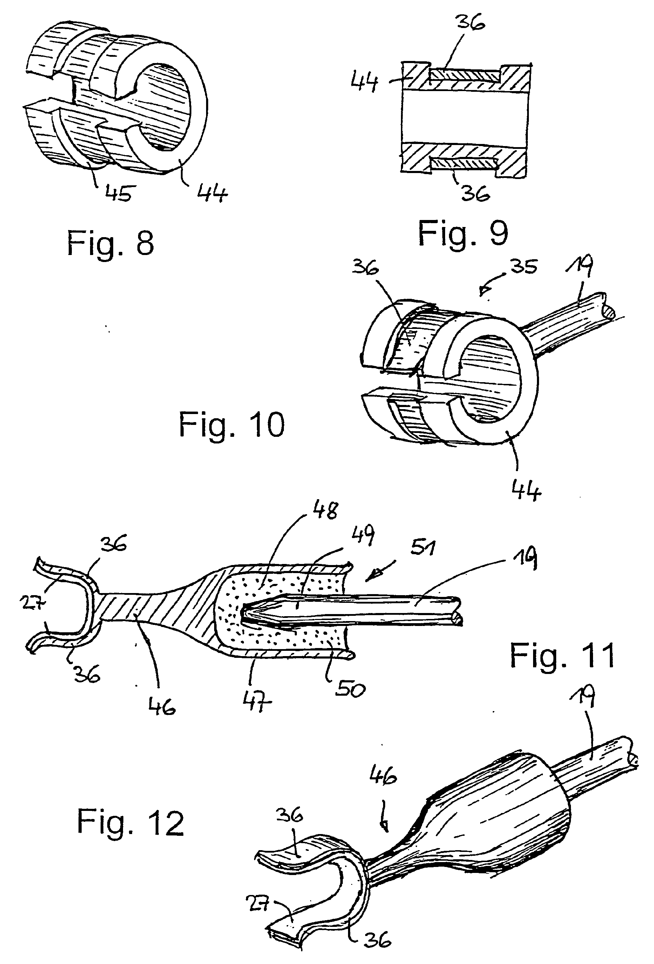

Die Figuren 8 bis 10 zeigen ein Ausführungsbeispiel eines Dämpfungsgliedes 44, das weitgehend der Ausführungsform gemäß den Figuren 6 und 7 entspricht. Bei dem das Dämpfungsglied 44 bildenden Formteil handelt es sich in diesem Fall um eine längsgeschlitzte Muffe oder Manschette aus entropie- oder gummielastischem Werkstoff, vorzugsweise Siliconharz. Diese Muffe wird ähnlich wie das Schlauchstück der Figuren 6 und 7 im Bereich der Ankoppelstelle 16 auf den betreffenden Teil der Ossikelkette aufgesteckt. Das Dämpfungsglied 44 weist eine zwischen den axialen Muffenenden befindliche Nut 45 auf, die sich in Umfangsrichtung erstreckt und in die sich die Federarme 36 des Koppelelements 35 einlegen. Die Nut 45 verhindert ein Abrutschen der Federarme 36 von dem Dämpfungsglied 44.

In den Figuren 11 und 12 ist ein weiteres Ausführungsbeispiel einer Anordnung veranschaulicht, bei welcher das Dämpfungsglied entsprechend Fig. 5 als Beschichtung 27 auf Federarmen 36 eines Koppelelements 46 ausgebildet ist. Das Koppelelement 46 ist im Bereich seines koppelstangenseitigen Endes 47 becherförmig gestaltet. In den dadurch geschaffenen Hohlraum 48 taucht das koppelelementseitige Ende 49 der Koppelstange 19 unter Bildung eines Spaltes 50 ein, ohne das Koppelelement 46 zu berühren. Der Spalt 50 kann mit einem entropie- oder gummielastischem Werkstoff gefüllt sein, welcher ein zusätzliches Dämpfungsglied 51 bildet. Es versteht sich, daß umgekehrt auch das koppelelementseitige Ende der Koppelstange als Becher ausgebildet sein kann, in den ein dem Stiel 26 des Koppelelements 22 oder ein dem Stiel 30 des Koppelelements 29 entsprechendes Teil des Koppelelements eintaucht.

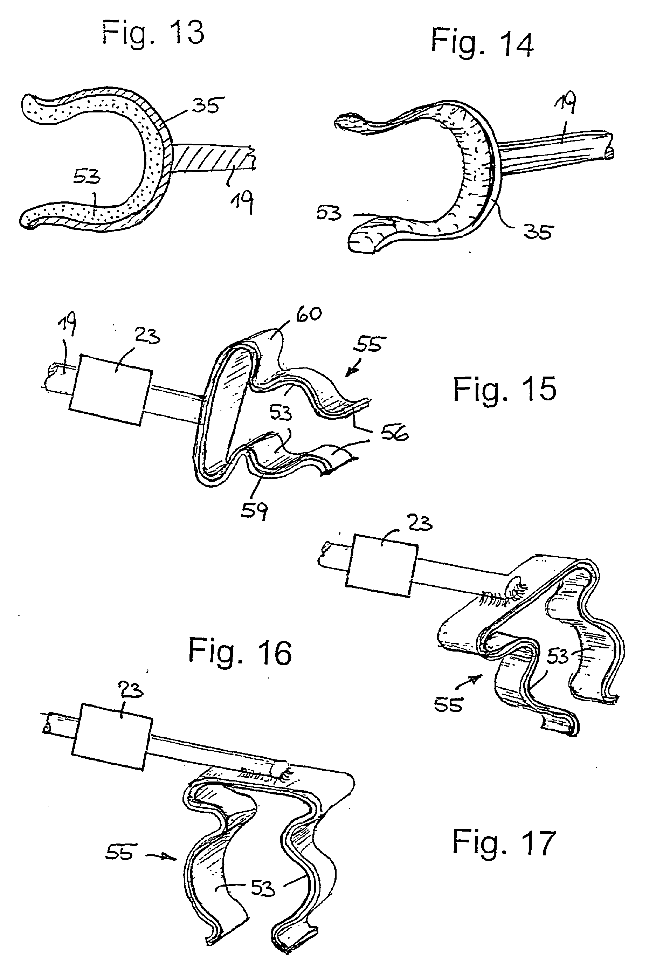

Auch bei der in den Figuren 13 und 14 gezeigten Ausführungsform ist als zwischen dem Koppelelement 35 und der Ankoppelstelle 16 liegendes Dämpfungsglied 53 eine Beschichtung mit entropieelastischen Eigenschaften vorgesehen. Die Beschichtung befindet sich wiederum auf der Seite des Koppelelements 35, die im implantierten Zustand mit der Ankoppelstelle 16 in Berührung kommt. Im Falle dieses Ausführungsbeispiels ist analog zu der Ausführungsform gemäß den Figuren 6 und 7 die Koppelstange 19 unmittelbar an dem Koppelelement 35 befestigt, zum Beispiel mit diesem verschweißt.

Die Figuren 15, 16 und 17 zeigen eine abgewandelte Form eines Koppelelements 55 in Form einer Federklammer mit zwei Federarmen 56. Die Federarme 56 unterscheiden sich von den Federarmen 36 im wesentlichen dadurch, daß sie zusätzlich zu dem einen Teil der Ossikelkette umgreifenden Abschnitt 59 einen gewellten Federabschnitt 60 aufweisen. Der Federabschnitt 60 verleiht dem Koppelelement 55 eine gewisse Energieelastizität, die vor allem für ein feinfühliges Ankoppeln an die empfindliche Struktur der Ossikelkette von Nutzen sein kann. Je nach Lage der Ankoppelstelle 16 können die Federarme 56 mit Bezug auf die Koppelstange 19 unterschiedlich ausgerichtet sein, wie dies aus den Figuren 15 bis 17 hervorgeht. Zwischen Koppelstange 19 und Koppelelement 55 kann in der skizzierten Weise die Kupplung 23 eingefügt sein. Auf mindestens den mit der Ankoppelstelle 16 in Berührung kommenden Teilen der Federarme 56 ist als Dämpfungsglied eine Beschichtung 53 mit entropieelastischen Eigenschaften entsprechend den Figuren 13 und 14 vorgesehen.

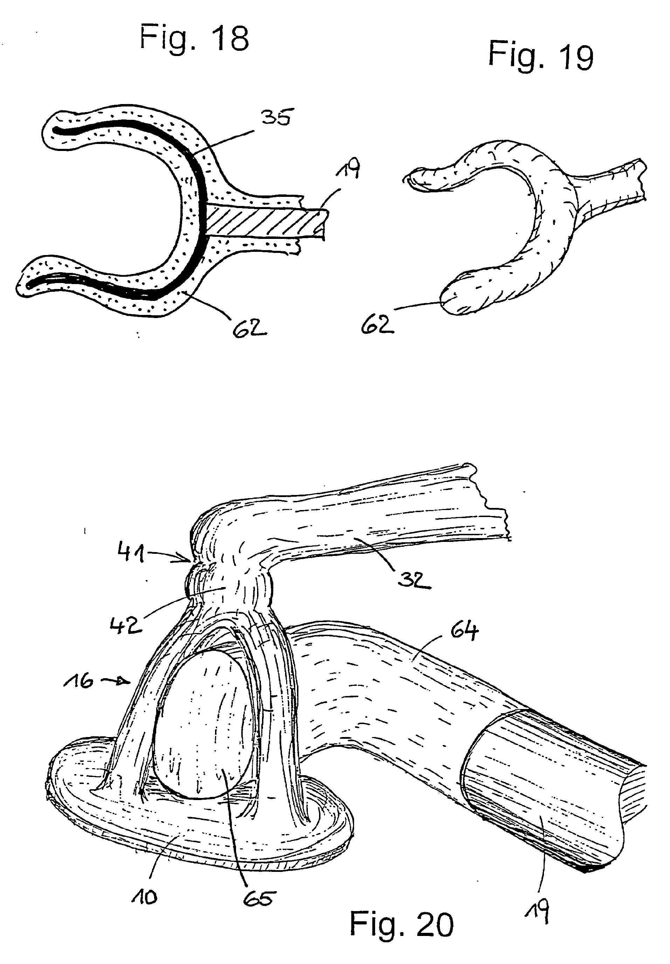

Im Falle des Ausführungsbeispiels der Figuren 18 und 19 ist das an der Koppelstange 19 befestigte Koppelelement 35 von einer Beschichtung 62 mit entropieelastischen Eigenschaften umhüllt. Die Beschichtung 62 kann beispielsweise durch eine Tauchsiliconisierung hergestellt sein, und sie bildet ein zwischen dem Koppelelement 35 und der Ankoppelstelle 16 liegendes Dämpfungsglied. Außerdem schützt die Beschichtung 62 den betreffenden Teil der empfindlichen Ossikelkette beim Ankoppelvorgang.

Die Fig. 20 zeigt eine Ausführungsform, bei der das Koppelelement selbst als Dämpfungsglied ausgebildet ist. Dabei ist an dem der Ankoppelstelle 16 zugewendeten Ende der Koppelstange 19 ein Formteil 64 aus Werkstoff mit entropieelastischen Eigenschaften, beispielsweise Siliconharz, befestigt. Das Formteil 64 kann, wie dargestellt, die Gestalt eines abgewinkelten Kreiszylinders haben; sein Durchmesser ist beispielsweise näherungsweise gleich dem Durchmesser der Koppelstange 19. Das freie Ende 65 des Formteils 64 wird beim Implantieren der Anordnung in den freien Raum zwischen dem Steigbügel 9 und der Steigbügelfußplatte 10 eingeschoben.

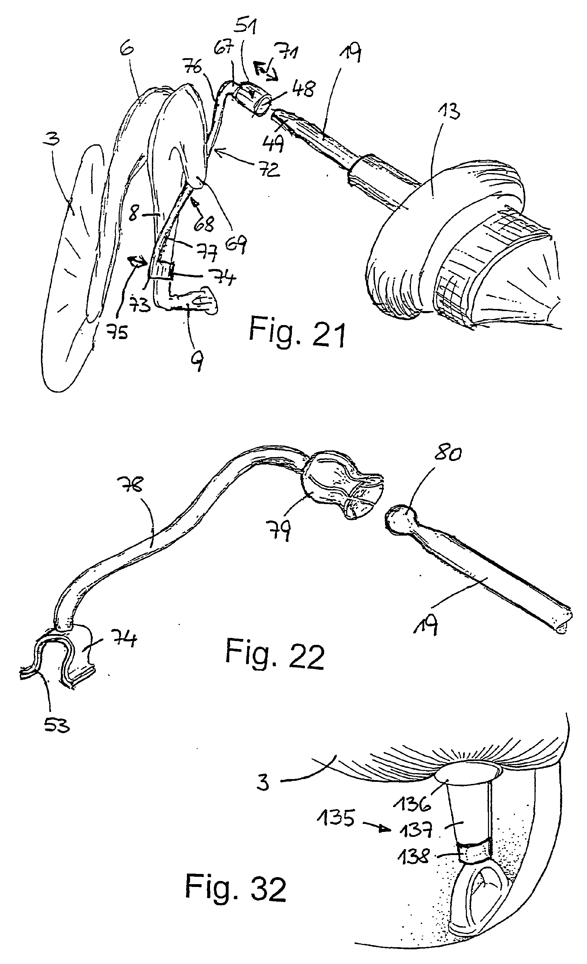

In Fig. 21 ist auseinandergezogen ein Ausführungsbeispiel dargestellt, bei dem im zusammengebauten Zustand das freie Ende 49 der Koppelstange 19 in einen Hohlraum 48 gemäß Fig. 11 eintaucht, der am koppelstangenseitigen Ende 67 eines Koppelelements 68 vorgesehen ist. Die Koppelstange 19 und das Koppelelement 68 sind über ein zusätzliches Dämpfungsglied 51 nach Art der Fig. 11 verbunden. Das Koppelelement 68 ist als zweiarmiger Hebel ausgebildet, der sich in einem mittleren Bereich an dem kurzen Amboßfortsatz 69 abstützt. Wird das koppelstangenseitigen Ende 67 mittels der Koppelstange 19 zu einer Bewegung entsprechend dem Doppelpfeil 71 veranlaßt, schwenkt das Koppelelement 68 um einen von dem kurzen Amboßfortsatz 69 bestimmten Drehpunkt 72. Dadurch wird das andere Ende 73 des Koppelelements 68, das mit dem langen Amboßfortsatz 8 über eine Federklammer 74 oder dergleichen und ein Dämpfungsglied in Form einer Beschichtung 53 (Figuren 13 und 14) oder 62 (Figuren 18 und 19) in Eingriff steht, gemäß dem Doppelpfeil 75 verstellt. Durch entsprechende Bemessung der relativen Längen der Arme 76 und 77 des Koppelelements 68 kann ein gewünschtes Hebelverhältnis eingestellt werden. Das Ausführungsbeispiel der Fig. 21 läßt sich unter anderem so abwandeln, daß eine feste Kopplung zwischen der Koppelstange 19 und dem Koppelelement 68 vorgesehen wird.

Im Falle der Ausführungsform gemäß Fig. 22 sitzt an einem koppelstangenseitigen Ende eines dem Koppelelement 68 ähnlichen Koppelelements 78 eine geschlitzte Kugelaufnahme 79. Die Koppelstange 19 trägt an ihrem koppelelementseitigen Ende einen Kugelkopf 80, der in die Kugelaufnahme 79 eingerastet wird und dann zusammen mit der Kugelaufnahme 79 ein Kugelgelenk bildet. Dieses Kugelgelenk läßt eine begrenzte Schwenkbewegung der Koppelstange 19 gegenüber dem Koppelelement 78 zu. Eine am anderen Ende des Koppelelements 78 befestigte Federklammer 74 ist zur Bildung eines Dämpfungsgliedes beispielsweise mit einer Beschichtung 53 der anhand der Figuren 13 und 14 erläuterten Art versehen.

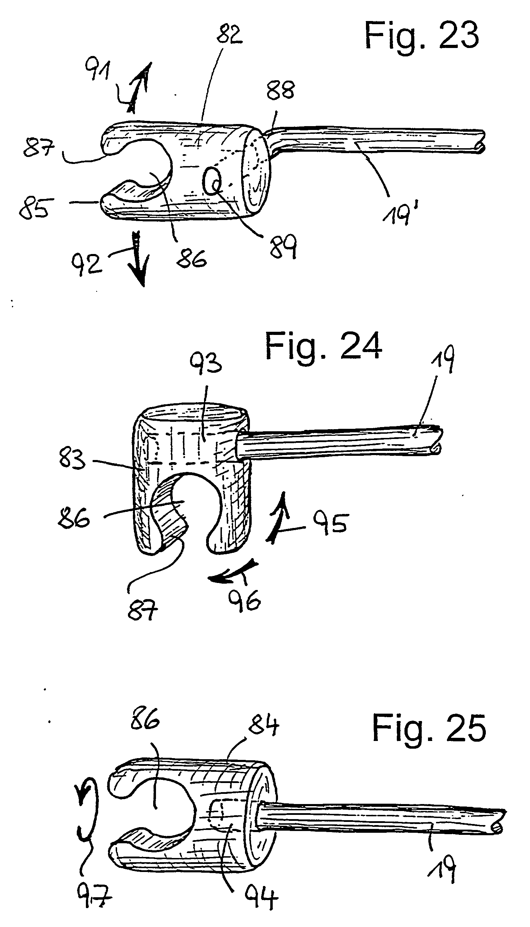

Die Figuren 23, 24 und 25 zeigen weitere Ausführungsbeispiele, von selbst ein Dämpfungsglied bildenden Koppelelementen 82, 83 beziehungsweise 84, die jeweils als Zylinder, zweckmäßig gerade Kreiszylinder, ausgebildet sind und aus einem Werkstoff mit entropieelastischen Eigenschaften, zum Beispiel Silicon, bestehen. Die Koppelelemente 82, 83 und 84 weisen nahe ihrem einen axialen Ende 85 jeweils eine senkrecht zu der Zylinderachse gerichtete Aufnahmeöffnung 86 für das Zielossikel auf. Die Aufnahmeöffnung 86 steht mit dem Zylinderende 85 über einen verengten Durchlaß 87 in Verbindung. Der Durchlaß 87 ist zum Zylinderende 85 hin vorzugsweise abgerundet, und er wird beim Aufschieben des Koppelelements auf das Zielossikel elastisch aufgeweitet.

Bei der Ausführungsform gemäß Fig. 23 ist eine Koppelstange 19' vorgesehen, die an ihrem koppelelementseitigen Ende 88 gekröpft ist. Das Koppelstangenende 88 greift in eine parallel zu der Aufnahmeöffnung 86 verlaufende Bohrung 89 des Koppelelements 82 ein, und zwar vorzugsweise mit einer solchen Passung, daß sich das Koppelelement 82 in Richtung der Pfeile 91 beziehungsweise 92 um das Koppelstangenende 88 schwenken läßt.

Die Ausführungsformen der Figuren 24 und 25 unterscheiden sich von der Ausführungsform gemäß Fig. 23 dadurch, daß eine gerade Koppelstange 19 vorgesehen ist. Das koppelelementseitige Ende der Koppelstange 19 greift im Falle der Fig. 24 in eine zu der Aufnahmeöffnung 86 und zu der Zylinderachse senkrecht gerichtete Bohrung 93 des Koppelelements 83 und im Falle der Fig. 25 in eine mit der Zylinderachse ausgerichtete Bohrung 94 des Koppelelements 84 ein. Vorzugsweise sind die Passungen wiederum so gewählt, daß sich die Koppelelemente 83, 84 in Richtung der Pfeile 95, 96 beziehungsweise 97 um das Koppelstangenende schwenken lassen.

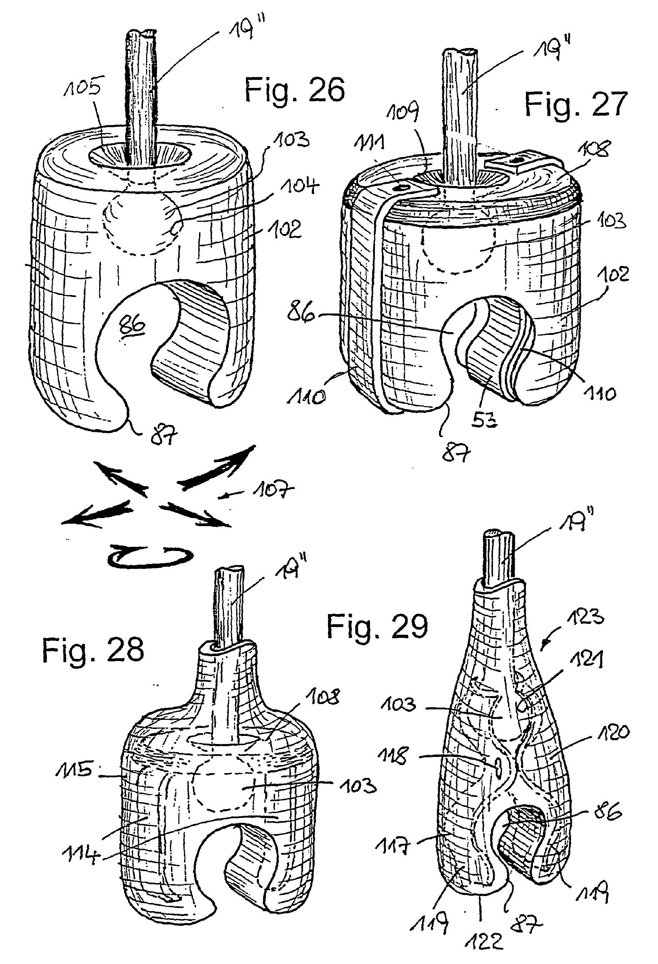

Auch bei den Ausführungsformen der Figuren 26 und 27 ist ein zylindrisches Koppelelement 102 vorgesehen, das selbst ein Dämpfungsglied bildet und aus einem Werkstoff mit entropieelastischen Eigenschaften, zum Beispiel vernetztem Silicon, gefertigt ist. Die Ausführungsform gemäß Fig. 26 ist weitgehend ähnlich dem Ausführungsbeispiel der Fig. 25. Die Koppelstange 19" ist dabei jedoch an ihrem koppelelementseitigen Ende mit einer Kugel 103 versehen, die in eine Kugelaufnahme 104 des Koppelelements 102 eingreift. Die Kugel 103 und die Kugelaufnahme 104 bilden gemeinsam ein Kugelgelenk. An die Kugelaufnahme 104 schließt in axialer Richtung des Koppelelements 102 eine sich nach außen trichterförmig erweiternde Ausnehmung 105 an. Diese Ausbildung gestattet es, das Koppelelement 102 in der durch die Pfeilgruppe 107 angedeuteten Weise in allen Raumrichtungen mit Bezug auf die Koppelstange 19'' zu schwenken und zu drehen.

Bei der Ausführungsform gemäß Fig. 27 trägt das Koppelelement 102 an der von dem Durchlaß 87 abliegenden Seite einen vorzugsweise metallischen Deckel 108. Der Dekkel 108 weist einen mit der Ausnehmung 105 ausgerichteten Durchbruch 109 auf, dessen Durchmesser kleiner als der Durchmesser der Kugel 103 ist. Der Deckel 108 bildet auf diese Weise einen Verlierschutz für die Kugel 103 und verhindert dadurch eine unbeabsichtigte Trennung von Koppelstange 19'' und Koppelelement 102. Des weiteren ist eine vorzugsweise metallische Federklammer 110 vorgesehen, welche das Koppelelement 102 auf diametral gegenüberliegenden Seiten umgreift und welche an den Stirnflächen des Koppelelements 102 sowie an gegenüberliegenden Innenseiten der Aufnahmeöffnung 86 und des Durchlasses 87 anliegt oder in diese Stirnflächen und/oder Innenseiten eingelassen ist. Die Enden der Federklammer 110 sind mit dem Deckel 108 verbunden, und zwar zweckmäßig bei 111 verschweißt. Mindestens der im implantierten Zustand an dem Zielossikel anliegende Teil der Oberfläche der Federklammer 110 ist mit einer Beschichtung 53 mit entropieelastischen Eigenschaften versehen. Diese Beschichtung bildet ein zusätzliches Dämpfungsglied mit Schutzfunktion für das Zielossikel.

Fig. 28 zeigt eine Ausführungsform ähnlich Fig. 27, bei der jedoch federnde, vorzugsweise metallische Bügel 114 in ein Koppelelement 115 aus einem Werkstoff mit entropieelastischen Eigenschaften, zum Beispiel Silicon, eingegossen sind. In dem veranschaulichten Ausführungsbeispiel umgreift der Werkstoff des als Dämpfungsglied ausgebildeten Koppelelements 115 auch einen Teil der Koppelstange 19'' auf der von der Kugel 103 abliegenden Seite des Deckels 108. Bei geeigneter Wahl dieses Werkstoffes und geeigneten Wandstärken im Koppelstangenbereich ist gleichwohl nicht nur ein Drehen des Koppelelements 115 um die Achse der Koppelstange 19'' möglich, sondern auch ein gegenseitiges Verschwenken von Koppelelement und Koppelstange. Die Bügel 114 sind an ihrem einen Ende zweckmäßig mit dem Deckel 108 verschweißt. Die Bügel 114 können aber auch Teil einer Federklammer sein, auf deren Mittelsteg der Deckel 108 aufgesetzt ist. Des weiteren können der Deckel und die Bügel einstückig miteinander verbunden sein.

Bei der abgewandelten Ausführungsform gemäß Fig. 29 ist als Koppelelement 117 eine federnde Klammer aus zwei bei 118 miteinander verschweißten gewellten Federarmen 119 vorgesehen. Das Koppelelement 117 ist in eine als Dämpfungsglied wirkende Umhüllung 120 aus einem Werkstoff mit entropieelastischen Eigenschaften, zum Beispiel Silicon, eingegossen. Die Federarme 119 bilden auf der einen Seite der Verbindungsstelle 118 eine Kugelaufnahme 121 für die Kugel 103 der Koppelstange 19" sowie auf der anderen Seite dieser Verbindungsstelle zusammen mit der Umhüllung 120 den aufspreizbaren Durchlaß 87 und die Aufnahmeöffnung 86 für das Zielossikel. Das Koppelelement 117 läßt sich zusammen mit der Umhüllung 120 gegenüber der Koppelstange 19'' drehen und schwenken. Der Durchlaß 87 befindet sich an der von der Koppelstange 19'' abgewendeten Stirnseite 122 des aus dem Koppelelement und seiner Umhüllung bestehenden Körpers 123.

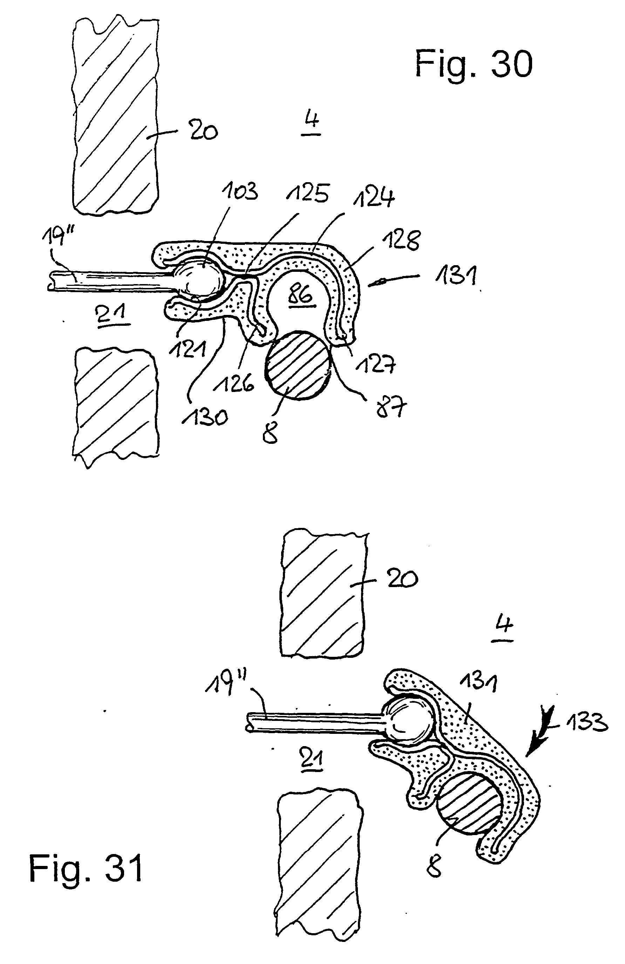

Die Ausführungsform gemäß den Figuren 30 und 31 ist weitgehend ähnlich derjenigen der Fig. 29. Auch in diesem Fall ist ein Koppelelement 124 vorgesehen, das von zwei bei 125 miteinander verbundenen, vorzugsweise verschweißten, Federarmen 126 und 127 gebildet wird, die in eine als Dämpfungsglied wirkende Umhüllung 128 aus einem Werkstoff mit entropieelastischen Eigenschaften, zum Beispiel Silicon, eingegossen sind. Die Federarme 126, 127 bilden die Kugelaufnahme 121 für die Kugel 103 der Koppelstange 19'' sowie zusammen mit der Umhüllung 128 den aufspreizbaren Durchlaß 87 und die Aufnahmeöffnung 86 für das Zielossikel 8. Abweichend von dem Ausführungsbeispiel der Fig. 29 ist der Durchlaß 87 an einer Seitenfläche 130 des aus dem Koppelelement 124 und seiner Umhüllung 128 bestehenden Körpers 131 angeordnet.

Der Körper 131 kann mittels der Koppelstange 19'' durch den Durchbruch 21 in der hinteren Gehörgangswand 20 hindurch in den Mittelohrraum 4 eingeführt und so positioniert werden, daß der aufspreizbare Durchlaß 87 mit dem Zielossikel, beispielsweise dem langen Amboßfortsatz 8, entsprechend Fig. 30 ausgerichtet ist. Dann wird der Körper 131 niedergedrückt und dadurch in Richtung des Pfeils 133 in Fig. 31 mit Bezug auf die Koppelstange 19'' geschwenkt, bis das Zielossikel unter Aufweitung des Durchlasses 87 in der Aufnahmeöffnung 86 liegt. Auf diese Weise wird eine sichere Ankopplung an das Zielossikel erreicht.

Als Werkstoff für die Koppelstange können alle bekannte biokompatible Metalle und ihren Legierungen verwendet werden, vor allem implantierfähiges Titan, insbesondere Reintitan mit einer Reinheit > 99,6 %. Daneben sind unter anderem Platin, Niob oder Tantal oder Legierungen von Titan, Platin, Niob beziehungsweise Tantal geeignet. Gegebenenfalls kann die Koppelstange aber auch aus einem implantierbaren keramischen Werkstoff, insbesondere Aluminiumoxid, bestehen. Die Federarme, Federklammern und Bügel der Koppelelemente können aus den gleichen Metallen und Metalllegierungen wie die Koppelstange gefertigt sein. Für die Koppelstange und die Koppelelemente können aber auch langzeit-implantierbare Kunststoffe vorgesehen sein, so unter anderem vernetzte Silicone, Polyurethane, PTFE, FEP, Polycarbonate und dergleichen, die gegebenenfalls faserverstärkt, insbesondere kohlefaserverstärkt, sein können.

In Fig. 32 ist schematisch ein implantiertes passives Hörsystem dargestellt, bei dem als zu mechanischen Schwingungen anregbares ausgangsseitiges Treiberteil das Trommelfell 3 genutzt ist. Am Trommelfell 3 liegt eine insgesamt mit 135 bezeichnete TORP-Prothese (total ossicular replacement prosthesis) mit einem Kopf 136 an, der eine abgerundete Oberfläche aufweist. An den Kopf 136 schließt ein Schaft 137 an, der mit dem Kopf 136 einstückig verbunden sein kann. Der Kopf 136 und der Schaft 137 bestehen zweckmäßig aus einem implantierbaren metallischen oder keramischen Werkstoff. Das von dem Kopf 136 abliegende Ende des Schafts 137 ist mit dem Steigbügelkopf 42 über ein Dämpfungsglied 138 gekoppelt, das entropieelastische Eigenschaften besitzt und vorzugsweise aus vernetztem Silicon gefertigt ist.

## Patentansprüche

1. Implantierbare Anordnung zum mechanischen Ankoppeln eines zu mechanischen Schwingungen anregbaren ausgangsseitigen Treiberteils (3, 15) eines aktiven oder passiven Hörsystems an eine vorgewählte Ankoppelstelle (16) an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran über eine Koppelanordnung (17), die ein mit der vorgewählten Ankoppelstelle (16) in Verbindung bringbares Koppelelement (22, 29, 35, 46, 55, 64, 68, 78, 82, 83, 84, 102, 115, 117, 124, 137) aufweist, **gekennzeichnet durch** ein im implantierten Zustand an der Ankoppelstelle (16) anliegendes Dämpfungsglied (27, 34, 44, 53, 62, 64, 82, 83, 84, 102, 115, 120, 128, 138) mit entropieelastischen Eigenschaften.

2. Anordnung nach Anspruch 1, **dadurch gekennzeichnet, daß** das Dämpfungsglied (27, 34, 44, 53, 62, 120, 128, 138) im implantierten Zustand mindestens zum Teil zwischen dem Koppelelement (22, 29, 35, 46, 55, 68, 78, 117, 124, 137) und der Ankoppelstelle (16) liegt.

3. Anordnung nach Anspruch 1 oder 2, **dadurch gekennzeichnet, daß** das Dämpfungsglied (27, 34, 44, 53, 62, 64, 82, 83, 84, 102, 115, 120, 128) so ausgebildet ist, daß es im implantierten Zustand einen Teil der Ossikelkette mindestens teilweise umgreift.

4. Anordnung nach Anspruch 2 oder 3, **dadurch gekennzeichnet, daß** das Dämpfungsglied (34, 44, 64, 82, 83, 84, 102, 115, 138) als Formteil ausgebildet ist.

5. Anordnung nach Anspruch 4, **dadurch gekennzeichnet, daß** das Dämpfungsglied (34) ein längsgeschlitztes Schlauchstück ist.

6. Anordnung nach Anspruch 4, **dadurch gekennzeichnet, daß** das Dämpfungsglied (44) eine längsgeschlitzte Muffe ist.

7. Anordnung nach Anspruch 6, **dadurch gekennzeichnet, daß** die Muffe (44) eine zwischen den axialen Muffenenden liegende, sich in Umfangsrichtung der Muffe erstreckende Nut (45) aufweist, in welche sich das Koppelelement (35) einlegt.

8. Anordnung nach Anspruch 2, **dadurch gekennzeichnet, daß** als Dämpfungsglied eine Beschichtung (27, 53, 62, 120, 128) mit entropieelastischen Eigenschaften auf der mit der Ankoppelstelle (16) in Berührung kommenden Seite des Koppelelements (22, 29, 35, 46, 55, 68, 78, 117, 124) vorgesehen ist.

9. Anordnung nach Anspruch 2, **dadurch gekennzeichnet, daß** das Dämpfungsglied als Beschichtung (62, 120, 128) mit entropieelastischen Eigenschaften ausgebildet ist, welche das Koppelelement (35, 117, 124) mindestens teilweise umschließt.

10. Anordnung nach Anspruch 1 oder 3, **dadurch gekennzeichnet, daß** das Koppelelement (64, 82, 83, 84, 102, 115) selbst als Dämpfungsglied ausgebildet ist.

11. Anordnung nach Anspruch 10, **dadurch gekennzeichnet, daß** als Koppelelement und Dämpfungsglied ein Formteil (64) mit entropieelastischen Eigenschaften vorgesehen ist, das mit einem freien Ende in den Raum zwischen Steigbügel und Steigbügelfußplatte einschiebbar ist.

12. Anordnung nach Anspruch 10 oder 11, **dadurch gekennzeichnet, daß** das als Dämpfungsglied ausgebildete Koppelelement (64, 82, 83, 84, 102, 115) mindestens näherungsweise zylindrische, vorzugsweise kreiszylindrische, Form hat.

13. Anordnung nach Anspruch 12, **dadurch gekennzeichnet, daß** in dem als Dämpfungsglied ausgebildeten Koppelelement (82, 83, 84, 102, 115) eine Aufnahmeöffnung (86) für ein Zielossikel (8) vorgesehen ist, in die das Zielossikel über einen aufspreizbaren Durchlaß (87) einbringbar ist.

14. Anordnung nach einem der vorhergehenden Ansprüche, **dadurch gekennzeichnet, daß** die Koppelanordnung (17) eine Koppelstange (19, 19', 19'') aufweist, mit welcher das Koppelelement (78, 82, 83, 84, 102, 115, 117, 124) drehbar und/oder schwenkbar verbunden ist.

15. Anordnung nach Anspruch 12, **dadurch gekennzeichnet, daß** die Koppelstange (19, 19', 19'') und das Koppelelement (78, 82, 83, 84, 102, 115, 117, 124) über ein Kugelgelenk (79, 80; 103, 104; 103, 121) miteinander verbunden sind.

16. Anordnung nach einem der vorhergehenden Ansprüche, **dadurch gekennzeichnet, daß** das Dämpfungsglied (27, 34, 44, 53, 62, 64, 82, 83, 84, 102, 115, 120, 128, 138) aus vernetztem Silicon oder einem anderen kautschukartigen Werkstoff besteht.

17. Anordnung nach einem der vorhergehenden Ansprüche, **dadurch gekennzeichnet, daß** sie Teil eines aktiven Hörsystems ist, bei dem das ausgangsseitige Treiberteil (15) ein schwingfähiges Teil eines elektromechanischen Hörgerätewandlers (13) ist.

18. Anordnung nach einem der vorhergehenden Ansprüche, **dadurch gekennzeichnet, daß** sie Teil eines passiven Hörsystems ist, bei dem im implantierten Zustand das Trommelfell als ausgangsseitiges Treiberteil genutzt ist.

## Claims

1. Implantable arrangement for mechanical coupling of an output driver part (3, 15) of an active or passive hearing system, the driver part being excitable to mechanical vibrations, to a pre-selected coupling site (16) to the ossicular chain, the foot plate of the stapes or a membrane which closes the round window or an artificial window in the cochlea, in the vestibulum or in the labyrinth (equilibrium organ), via a coupling arrangement (17) which has a coupling element (22, 29, 35, 46, 55, 64, 68, 78, 82, 83, 84, 102, 115, 117, 124, 137) which can be connected to the pre-selected coupling site (16), **characterized by** an attenuator (27, 34, 44, 53, 62, 64, 82, 83, 84, 102, 115, 120, 128, 138) with entropy-elastic properties that in the implanted state, rests against the pre-selected coupling site (16).

2. Arrangement according to claim 1, **characterized in that** the attenuator (27, 34, 44, 53, 62, 120, 128, 138), in the implanted state, is positioned at least partially between the coupling element (22, 29, 35, 46, 55, 68, 78, 117, 124, 137) and the coupling site (16).

3. Arrangement according to claim 1 or 2, **characterized in that** the attenuator (27, 34, 44, 53, 62, 64, 82, 83, 84, 102, 115, 120, 128) is constructed to at least partially surround part of the ossicular chain in the implanted state.

4. Arrangement according to claim 2 or 3, **characterized in that** the attenuator (34, 44, 64, 82, 83, 84, 102, 115, 138) is formed as a moulded part.

5. Arrangement according to claim 4, **characterized in that** the attenuator (34) is a longitudinally slotted piece of tubing.

6. Arrangement according to claim 4, **characterized in that** the attenuator (44) is a longitudinally slotted sleeve.

7. Arrangement according to claim 6, **characterized in that** the sleeve (44) has a groove (45) which is located between the axial ends of the sleeve and which extends in the circumferential direction of the sleeve, into which groove the coupling element (35) inserts.

8. Arrangement according to claim 2, **characterized in that** the attenuator is provided as a coating (27, 53, 62, 120, 128) with entropy-elastic properties on the side of the coupling element (22, 29, 35, 46, 55, 68, 78, 117,124) that comes into contact with the coupling site (16).

9. Arrangement according to claim 2, **characterized in that** the attenuator is provided as a coating (62, 120, 128) with entropy-elastic properties which at least partially surrounds the coupling element (35, 117, 124).

10. Arrangement according to claim 1 or 3, **characterized in that** the coupling element (64, 82, 83, 84, 102, 115) itself is formed as attenuator.

11. Arrangement according to claim 10, **characterized in that** a moulded part (64) having entropy-elastic properties is provided as coupling element and attenuator, a free end of which is insertable into the space between the stapes and the foot plate of the stapes.

12. Arrangement according to claim 10 or 11, **characterized in that** the coupling element (64, 82, 83, 84, 102,115) formed as attenuator has an at least roughly cylindrical, preferably circular cylindrical shape.

13. Arrangement according to claim 12, **characterized in that** in the coupling element (82, 83, 84, 102, 115) formed as attenuator a receiving opening (86) for a target ossicle (8) is provided into which the target ossicle is insertable via a spreadable passage (87).

14. Arrangement according to anyone of the preceding claims, **characterized in that** the coupling arrangement (17) has a coupling rod (19, 19', 19") to which the coupling element (78, 82, 83, 84, 102, 115, 117, 124) is connected in a rotatable and/or swivable manner.

15. Arrangement according to claim 14, **characterized in that** the coupling rod (19, 19', 19") and the coupling element (78, 82, 83, 84, 102, 115, 117, 124) are connected to each other by means of a ball-and-socket joint (79, 80; 103, 104; 103, 121).

16. Arrangement according to anyone of the preceding claims, **characterized in that** the attenuator (27, 34, 44, 53, 62, 64, 82, 83, 84, 102, 115, 120, 128, 138) is made of crosslinked silicon or another natural rubber-like material.

17. Arrangement according to any one of the preceding claims, **characterized in that** it forms part of an active hearing system, in which the output driver part (15) is a vibratable part of an electro-mechanical hearing aid converter (13).

18. Arrangement according to any one of claims 1 to 16, **characterized in that** it is part of a passive hearing system, in which, in the implanted state, the ear drum is used as the output driver part.

## Revendications

1. Dispositif implantable destiné au couplage mécanique d'un élément de stimulation (3, 15), pouvant être excité en vibrations mécaniques, situé du côté de la sortie d'un système auditif actif ou passif, au niveau d'un emplacement de couplage (16) présélectionné de la chaîne des osselets, de la platine de l'étrier ou d'une membrane fermant la fenêtre ronde ou une fenêtre artificielle dans la cochlée, le vestibule ou le labyrinthe (organe d'équilibre), par l'intermédiaire d'un système de couplage (17) comportant un élément de couplage (22, 29, 35, 46, 55, 64, 68, 78, 82, 83, 84, 102, 115, 117, 124, 137) pouvant être relié à l'emplacement de couplage (16) présélectionné, **caractérisé par** un élément d'amortissement (27, 34, 44, 53, 62, 64, 82, 83, 84, 102, 115, 120, 128, 138) à propriétés élastiques entropiques, s'appliquant à l'état implanté contre l'emplacement de couplage (16).

2. Dispositif selon la revendication 1, **caractérisé en ce qu'**à l'état implanté, l'élément d'amortissement (27, 34, 44, 53, 62, 120, 128, 138) est situé, au moins en partie, entre l'élément de couplage (22, 29, 35, 46, 55, 68, 78, 117, 124, 137) et l'emplacement de couplage (16).

3. Dispositif selon la revendication 1 ou 2, **caractérisé en ce que** l'élément d'amortissement (27, 34, 44, 53, 62, 64, 82, 83, 84, 102, 115, 120, 128) est agencé de telle sorte qu'à l'état implanté, il entoure au moins partiellement une partie de la chaîne des osselets.

4. Dispositif selon la revendication 2 ou 3, **caractérisé en ce que** l'élément d'amortissement (34, 44, 64, 82, 83, 84, 102, 115, 138) est réalisé en tant que pièce de forme.

5. Dispositif selon la revendication 4, **caractérisé en ce que** l'élément d'amortissement (34) est une pièce flexible fendue longitudinalement.

6. Dispositif selon la revendication 4, **caractérisé en ce que** l'élément d'amortissement (44) est un manchon fendu longitudinalement.

7. Dispositif selon la revendication 6, **caractérisé en ce que** le manchon (44) comporte une rainure (45) située entre les extrémités axiales du manchon, qui s'étend dans la direction périphérique du manchon, dans laquelle s'engage l'élément de couplage (35).

8. Dispositif selon la revendication 2, **caractérisé en ce qu'**un revêtement (27, 53, 62, 120, 128) à propriétés élastiques entropiques est prévu en tant qu'élément d'amortissement sur la face entrant en contact avec l'emplacement de couplage (16) de l'élément de couplage (22, 29, 35, 46, 55, 68, 78, 117, 124).

9. Dispositif selon la revendication 2, **caractérisé en ce que** l'élément d'amortissement est réalisé en tant que revêtement (62, 120, 128) à propriétés élastiques entropiques, qui entoure au moins partiellement l'élément de couplage (35, 117, 124).

10. Dispositif selon la revendication 1 ou 3, **caractérisé en ce que** l'élément de couplage (64, 82, 83, 84, 102, 115) est lui-même réalisé en tant qu'élément d'amortissement.

11. Dispositif selon la revendication 10, **caractérisé en ce que**, en tant qu'élément de couplage et élément d'amortissement, il est prévu une pièce de forme (64) à propriétés élastiques entropiques, qui peut être insérée par son extrémité libre dans l'espace situé entre l'étrier et la platine de l'étrier.

12. Dispositif selon la revendication 10 ou 11, **caractérisé en ce que** l'élément de couplage (64, 82, 83, 84, 102, 115) réalisé en tant qu'élément d'amortissement présente au moins approximativement une forme cylindrique, de préférence cylindrique circulaire.

13. Dispositif selon la revendication 12, **caractérisé en ce que**, dans l'élément de couplage (82, 83, 84, 102, 115) réalisé en tant qu'élément d'amortissement, il est prévu un orifice de réception (86) pour un osselet cible (8), dans lequel l'osselet cible peut être introduit par l'intermédiaire d'un passage (87) susceptible de s'élargir.

14. Dispositif selon l'une des revendications précédentes, **caractérisé en ce que** le système de couplage (17) comporte une tige de couplage (19, 19', 19"), avec laquelle l'élément de couplage (78, 82, 83, 84, 102, 115, 117, 124) est relié en rotation et/ou en pivotement.

15. Dispositif selon la revendication 14, **caractérisé en ce que** la tige de couplage (19, 19', 19") et l'élément de couplage (78, 82, 83, 84, 102, 115, 117, 124) sont reliés entre eux par une rotule (79, 80 ; 103, 104 ; 103, 121).

16. Dispositif selon l'une des revendications précédentes, **caractérisé en ce que** l'élément d'amortissement (27, 34, 44, 53, 62, 64, 82, 83, 84, 102, 115, 120, 128, 138) est constitué de silicone réticulée ou d'un autre matériau de type caoutchouc.

17. Dispositif selon l'une des revendications précédentes, **caractérisé en ce qu'**il fait partie d'un système auditif actif, dans lequel l'élément de stimulation (15) situé du côté de la sortie est un élément susceptible de vibrer d'un convertisseur électromécanique (13) d'appareil auditif.

18. Dispositif selon l'une des revendications 1 à 16, **caractérisé en ce qu'**il fait partie d'un système auditif passif, dans lequel le tympan est utilisé à l'état implanté en tant qu'élément de stimulation situé du côté de la sortie.
